# EUROPÄISCHE PATENTSCHRIFT

(11) **EP 0 918 750 B1**
(45) Veröffentlichungstag und Bekanntmachung des Hinweises auf die Patenterteilung: **14.08.2002**
(21) Anmeldenummer: 97924982.8
(22) Anmeldetag: 23.05.1997
(51) Int. Cl.: C07C 281/18, A61K 31/655, C07D 209/14

(54) **NEUE GUANIDINDERIVATE, VERFAHREN ZU IHRER HERSTELLUNG UND IHRE VERWENDUNG ALS ARZNEIMITTEL**
NEW GUANIDINE DERIVATIVES, METHODS OF PREPARING THEM AND THEIR USE AS DRUGS
NOUVEAUX DERIVES DE GUANIDINE, PROCEDES PERMETTANT DE LES PREPARER ET LEUR UTILISATION COMME MEDICAMENTS

(30) Priorität: 24.05.1996 DE 19621038
(43) Veröffentlichungstag der Anmeldung: 02.06.1999
(73) Patentinhaber: Cancer Research Ventures Limited, London WC1E 7EB (GB)
(72) Erfinder: AMTMANN, Eberhard, D-69121 Heidelberg (DE); FRANK, Norbert, D-69118 Heidelberg (DE); SAUER, Gerhard, D-69118 Heidelberg (DE); SCHILLING, Gerhard, D-68526 Ladenburg (DE)
(74) Vertreter: Cripps, Joanna Elizabeth
(86) Internationale Anmeldenummer: EP9702658
(87) Internationale Veröffentlichungsnummer: WO9745401

(56) Entgegenhaltungen:
- GB-A- 932 951
- US-A- 3 914 308
- PATENT ABSTRACTS OF JAPAN vol. 007, no. 086 (C-161), 9.April 1983 & JP 58 015913 A (MITSUBISHI KASEI KOGYO KK), 29.Januar 1983,
- JERUSHALMY, Z. ET AL: "Inhibition by guanidino compounds of platelet aggregation induced by adenosine diphosphate" BIOCHEM. PHARMACOL. (1966), 15(11), 1791-803 CODEN: BCPCA6, 1966, XP002042926
- CHEMICAL ABSTRACTS, vol. 83, no. 7, 18.August 1975 Columbus, Ohio, US; abstract no. 53185, CARLSSON, FRITZ H. H. ET AL: "Biological activity of some guanylhydrazones and thiosemicarbazones of aliphatic carbonyl compounds" XP002042934 & CARBOHYDR. RES. (1974), 36(2), 359-68 CODEN: CRBRAT, 1974,
- K.C. AGRAWAL ET AL : "Potential antitumor agents. II. Effects of modifications in the side chain of 1-formylisoquinoline thiosemicarbazone." JOURNAL OF MEDICINAL CHEMISTRY., Bd. 12, Nr. 5, 1969, WASHINGTON US, Seiten 771-774, XP002042927
- B.S. PITZELE ET AL : "Potential antisecretory antidiarrheals. 1. alpha2-adrenergic aromatic aninoguanidine hydrazones." JOURNAL OF MEDICINAL CHEMISTRY., Bd. 31, 1988, WASHINGTON US, Seiten 138-144, XP002042928
- MARTIN, ELISABETH ET AL: "Basicity of amidino hydrazone derivatives" PHARMAZIE (1985), 40(5), 356-7 CODEN: PHARAT;ISSN: 0031-7144, 1985, XP002042929
- JIRA, T. ET AL: "HPLC studies on the distribution behavior of guanylic and N-phenylguanylic hydrazone derivatives" PHARMAZIE (1985), 40(1), 34-6 CODEN: PHARAT;ISSN: 0031-7144, 1985, XP002042930
- PRASAD, RAJ N. ET AL: "Acylation of guanidines and guanylhydrazones" CAN. J. CHEM. (1967), 45(19), 2247-52 CODEN: CJCHAG, 1967, XP002042931
- W. O. FOYE ET AL: "Synthesis and biological activity of guanylhydrazones of 2- and 4-pyridine and 4-quinoline carboxaldehydes." JOURNAL OF PHARMACEUTICAL SCIENCES, Bd. 79, Nr. 6, Juni 1990, Seiten 527-530, XP002042932
- JENEY, ENDRE ET AL: "Molluscacidal activities of organic bases and their salts" ZENTRALBL. BAKTERIOL., PARASITENKD., INFEKTIONSKR. HYG., ABT. 1 ORIG. (1967), 202(4), 539-46 CODEN: ZBPHA6, 1967, XP002042933

## Beschreibung

Die vorliegende Erfindung betrifft neue Guanidinderivate, Verfahren zu ihrer Herstellung und ihre Verwendung als Sphingomyelinase-Hemmstoffe und Arzneimittel, die diese Verbindungen enthalten.

Die Guanidinderivate der vorliegenden Erfindung entsprechen der allgemeinen Formel I:

In der GB 0 932 951 wird offenbart, daß Alkyliden- und Alkenyliden-aminoguanidine der allgemeinen Formel: R₁-C(R₂)=N-NH-C(NH₂)=NH, worin R₁ eine Alkyl- oder Alkenylgruppe mit fünf bis siebzehn Kohlenstoffatomen bedeutet, von denen nicht mehr als dreizehn in einer geraden Kette liegen, und R₂ ein Wasserstoffatom oder eine gerade bzw. verzweigte Alkylgruppe enthaltend von ein bis fünf Kohlenstoffatome bedeutet, sowie Säureanlagerungssalze davon, wirksam sind gegen Pilzen, die eine pathogenische Wirkung gegenüber Pflanzen aufweisen.

Die US 3,914,308 offenbart ein Verfahren zur Umsetzung von N-(2-alkylidin)-aminoguanidine, die 7 bis 15 Kohlenstoffatome im Alkylrest enthalten und die normalerweise in Petroleumöl unlöslich sind, in lösliche Komplexe durch Behandlung mit alkylierten Phenolen, sowie die öl-löslichen Komplexe, die darin resultieren. Ferner offenbart dieses Patent, daß diese N-(2-alkylidin)-aminoguanidine eine pestizidische, insbesondere befallverhütende, Aktivität aufweisen.

In Biochemical Pharmacology (1996) 15: 1791-1803, wurden verschiedene Guanidinound verwandte Verbindungen getestet, um ihre Fähigkeit zur Hemmung von Adenosindiphosphät-induzierte Plättchenaggregation zu bestimmen. Weiterhin wurde der Einfluß der aktiveren Verbindungen auf mehrere Plättchenfunktionen sowie auf die thrombische Aktivität bestimmt. Einige Aminoalkylguanidine, Alkylendiguanidine und substituierte Alkylendiguanidine haben sich als Hemmungsmittel erwiesen.

Die vorliegende Erfindung versieht neue Guanidinderivate, wie sie in Anspruch 1 definiert werden. Ferner vorsieht die Erfindung die Guanidinderivate, wie sie in Anspruch 4 definiert werden, zusammen mit pharmazeutisch verträglichen Bindemitteln und Trägerstoffen; weiterhin vorsieht die Erfindung diese Guanidinderivate zur Verwendung in Methoden der medizinischen Behandlung.
Die Guanidinderivate der vorliegenden Erfindung entsprechen der allgemeinen Formel I: 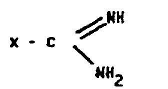 worin X -NH-NH-CH₂R₁ und -NH-N=CHR₁
R₁ C₈ - C₂₀ - Alkyl - verzweigt oder unverzweigt-
bedeuten kann -
gegebenenfalls in Form der einzelnen optischen Isomeren, Mischungen der einzelnen Isomeren oder Racemate, tautomere bzw. geometrische Isomere - wie z.B. cis/trans-Isomere sowie in Form der freien Basen oder der entsprechenden Säureadditionsalze mit pharmakologisch unbedenklichen Säuren.

Besonders bevorzugt sind Verbindungen der allgemeinen Formel I, worin
- X -: NH-NH-CH₂R₁ und -NH-N=CHR₁. und
- R₁: einen unverzweigten Decylrest bedeutet

Die erfindungsgemäßen Verbindungen besitzen wertvolle pharmakodynamische und biochemische Eigenschaften und können deshalb in der Forschung und in der Human- und Tiermedizin vorteilhaft angewandt werden.

Überraschenderweise wurde nämlich gefunden, daß die erfindungsgemäßen Aminoguanidine und Amidine vorteilhafte Sphingomyelinase-hemmende, antimikrobielle, antivirale, antiinflammatorische (z.B. Anti-Schock-) und das Zellwachstum beeinflussende Wirkungen aufweisen.

Die Herstellung der erfindungsgemäßen Verbindungen erfolgt durch Umsetzung eines Aldehyds oder Ketons der Formel R₁CHO mit Aminoguanidin. Die Umsetzung wird üblicherweise in einem inerten organischen Lösungsmittel durchgeführt, beispielsweise einem chlorierten Kohlenwasserstoff, wie Dichlormethan oder Chloroform, oder einem aromatischen Kohlenwasserstoff, wie Benzol oder Toluol. Die Umsetzung wird zweckmäßigerweise so durchgeführt, daß das gebildete Wasser aus dem Gleichgewicht entfernt wird, beispielsweise mit Hilfe eines Wasserabscheiders. Die Umsetzung kann in einem breiten Temperaturbereich durchgeführt werden, im allgemeinen arbeitet man jedoch bei erhöhter Temperatur, insbesondere bei einer Temperatur im Bereich von etwa 60°C bis zum Siedepunkt des Reaktionsgemisches. Daneben können die erfindungsgemäßen Verbindungen nach an sich aus dem Stand der Technik bekannten Verfahren hergestellt werden. Die Ausgangsverbindungen sind bekannt oder können nach bekannten Methoden hergestellt werden.

Die erfindungsgemäßen Arzneimittel enthalten eine der oben genannten Verbindungen der allgemeinen Formel I in einem üblichen festen oder flüssigen Arzneimittelträger. Die erfindungsgemäßen Verbindungen können auch mit bekannten Wirkstoffen kombiniert werden.

Die erfindungsgemäßen Verbindungen zeichnen sich durch antiinflammatorische (z.B. Anti-Schock-), antimikrobielle, antitumorale und insbesondere antivirale Wirkungen aus. Das antivirale Wirkungsspektrum umfaßt z.B. Herpes-, Vesikular Stomatitis-, HIV- und Papillomaviren. Außerdem wurde gefunden, daß die erfindungsgemäßen Verbindungen das Wachstum von Tumorzellen beeinflussen. Es können Karzinome, z.B. Dickdarmkarzinom, Sarkome oder Leukämien behandelt werden.

Generell kann festgestellt werden, daß diese erfindungsgemäßen Substanzen eine NF-kappaB-abhängige Immunsuppression bewirken.

Die erfindungsgemäßen Verbindungen sind demgemäß zur Behandlung folgender Erkrankungen einsetzbar:
A. Systemische Entzündungsreaktionen
   Sepsis-verursachende Erkrankungen
   grampositive Sepsis
   gramnegative Sepsis
   Pilzsepsis
   Agranulozytose (neutropenic fever)
   Hamwegsinfektionen (Urosepsis)
   Allgemeininfektionen mit Meningokokken (meningococcemia)
   Traumen/Blutungen
   Verbrennungen
   Schädigungen hervorgerufen durch ionisierende Strahlung
   Akute Pancreatitis
   Schocklunge, (Adult respiratory distress syndrome, ARDS)
B. Reperfusionssyndrome
   Post pump syndrome
   Ischämie-induzierte Reperfusionschäden
C. Kardiovaskuläre Erkrankungen:
   Cardiac stun syndrome
   Myokardialer Infarkt
   Congestive heart failure
   Arteriosklerose
D. Infektionskrankheiten:
   Papilloma Virus Infektionen
   Herpes Virus Infektionen
   HIV Infektion/HIV Neuropathologie
   Meningitis
   Hepatitis
   Septische Arthritis
   Peritonitis
   Lungenentzündung
   Bronchitis
   Epiglottitis
   E. coli 0157:H7 Infektion
   Hemolytic uremic syndrome/thrombolytic thrombocytopenic purpura
   Malaria
   Hemorrhagisches Dengue Fieber
   Leishmaniasis
   Lepra
   Toxischer Schock
   Streptococcen Myositis
   Gasbrand
   Mycobacterium Tuberculose Infektionen
   Mycobacterium avium intracellulare Infektionen
   Pneumocystosis
   Pelvic inflammatory disease
   Orchitis/Epidydimitis
   Legionellosis
   Lyme Krankheit
   Influenza A Virus Infektion
   Erkrankungen hervorgerufen durch Epstein-Barr Virus
   Viral-associated hemaphagocytic syndrome
   Virale Enzephalitis/aseptische Meningitis
E. Gynäkologische Anwendungen
   Frühgeburt
   Fehlgeburt
   Infertilität
F. Entzündliche Erkrankungen / Autoimmunerkrankungen:
   Rheumatoide Arthritis / Seronegative Arthropathie
   Emphysembronchitis, (Chronic obstructive pulmonary disease,COPD)
   Osteoarthritis
   Entzündliche Darmerkrankungen
   Morbus Crohn
   Systemischer Lupus Erythematosis
   Iridocyclitis / Uveitis / optic neuritis
   Idiopathische pulmonare Fibrosis
   Systemische Vasculitis/Wegner's Granulomatose
   Sarcoidosis
   Orchitis/vasectomy reversal procedures
H. Allergische / atopische Erkrankungen:
   Asthma
   Allergische Rhinitis
   Ekzem Erkrankungen
   Allergische Kontaktdermatitis
   Allergische Konjunctivitis
   Hypersensitive pneumonitis
I. Maligne Erkrankungen:
   Tumortherapie in Kombination mit Chemotherapie, Radiotherapie und
   Zytokinbehandlung, wie z.B.:TNF-α Behandlung von Sarkomen,
   Karzinomen und Leukämien
   ALL
   AML
   CML
   CLL
   Mamakarzinome
   kleinzelliges und nicht-kleinzelliges Bronchialkarzinom
   Plattenepithelkarzinom
   Morbus Hodgkins, non-Hodgkin's Lymphom
   Multiples Melanom
   Kaposi Sarkom
   Colorectal Karzinom
   Nasopharyngeal Karzinom
   Maligne Histiocytosis
   Paraneoplastische syndrome/Hypercalcaemie malignen Charakters
J. Transplantations-Komplikationen
   Abstoßungsreaktionen nach Transplantationen
   Graft-versus-host Reaktionen
K Kachexie
L. Angeborene Erkrankungen:
   Cystische Fibrose
   Familial hematophagocytic lymphohistiocytosis
   Sichelzellen Anämie
M. Hauterkrankungen:
   Psoriasis
   Alopecea
N. Nervenerkrankungen/chronische und akute Neurodegenerationen
   Multiple Sklerose
   Morbus Alzheimer
   Morbus Parkinson
   Down's Syndrom
   Schlaganfall
   Schädel-Him-Trauma
   Migräne
O. Erkrankungen der Niere:
   Nephrotisches Syndrom
   Haemodialyse
   Urämie
P. Verschiedenste Intoxikationen:
   OKT3-Therapie
   Anti-CD3-Therapie
   Cytokin-Therapie
   Chemotherapie
   Bestrahlungstherapie
   Chronische Salicylat-Intoxikation
Q. Metabolische/idiopathische Erkrankungen:
   Morbus Wilson
   Hemachromatose
   Alpha-1 -Antitrypsin-Mangel
   Diabetes
   Hashimoto's Thyroiditis
   Osteoporose
   Hypothalamic-pituitary-adrenal axis evaluation
   Primary biliary cirrhosis

Durch in vitro Untersuchungen in Plaque-Reduktionstests unter Verwendung verschiedener Viren wurde eine Wachstumshemmung bei Substanzkonzentrationen von 0,1 bis 1000 /µg/ml festgestellt. Die Toxizität der erfindungsgemäßen Substanzen ist verhältnismäßig gering. Sie können vor allem als wirksame Prophylaktika oder Therapeutika gegen Grippe, Aids oder Herpes-Erkrankungen der Haut und Schleimhäute verwendet werden. Die Tagesdosis für Erwachsene während der Zeit der Erkrankung liegt in der Größenordnung von etwa 5 bis 1000 mg Wirkstoff täglich.

Die Verabreichung der erfindungsgemäßen Verbindungen kann parenteral, subkutan, intravenös, intramuskulär und intraperitoneal erfolgen. In diesem Fall stellt die Trägersubstanz eine sterile Flüssigkeit dar, wie Wasser oder Öl, wobei pflanzliche, tierische oder synthetische Öle eingesetzt werden. Als. Injektionslösungen dienen gewöhnlich Glukoselösungen. Die flüssigen Träger der injizierbaren Lösungen enthalten im allgemeinen 0,5 bis 26 Gew.-% Wirksubstanz. Mit gleichem Erfolg können die erfindungsgemäßen Verbindungen oral verabreicht werden. Ebenso eignen sich die Verbindungen für die Behandlung von Pneumonien in Form von Dampf oder Spray im Mundund Nasenraum. Für die orale Verabreichung kommen in erster Linie Zusammensetzungen in Form von Tabletten, Kapseln, Pulvern, Lösungen, Suspensionen oder Elixieren in Betracht. Die Menge des aktiven Bestandteils beträgt in diesen Verabreichungen wenigstens 1 Gew.-% bezogen auf das Gesamtgewicht der Zusammensetzung. Topisch können die erfindungsgemäßen Wirkstoffe ebenfalls appliziert werden, z.B. in Salben, Cremes, Emulsionen oder Lotionen

Die nachstehenden Beispiele zeigen einige Möglichkeiten für die Formulierung der Darreichungsformen auf:

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die erfindungsgemäßen Verbindungen sind ausgehend von aus dem Stand der Technik bekannten Verbindungen u.a. nach den in den folgenden Beispielen beschriebenen Verfahren herstellbar. Verschiedenartige, andere Ausgestaltungen der Erfindung sowie der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind. Ergänzend wird auf die Deutsche Patentanmeldung P 196 21 038.0 verwiesen auf die hiermit vollinhaltlich Bezug genommen wird.

### Beispiel 1

### Herstellung von 1-(Undecylidenamino)guanidin [C11AG]

1 mol (170.3 g) Undecanal, 1,1 mol (150 g) Aminoguanidinhydrogencarbonat und 1 g p-Toluolsulfonsäure werden mit 500 ml Toluol versetzt und unter Rühren zum Rückfluß erhitzt. Sobald 2 mol Wasser am Wasserabscheider abgeschieden sind, läßt man abkühlen, engt am Rotationsverdampfer ein und nimmt das dunkelrote Öl in 250 ml Petroläther 40/60° auf. Der dabei entstehende Niederschlag wird abgesaugt und nochmals mit Petroläther gewaschen. Zum Umkristallisieren wird der Niederschlag in Essigsäureethylester gelöst und in der Siedehitze mit Petroläther (Siedebereich 40 bis 60°C) bis zur beginnenden Trübung versetzt. Man erhält feine Kristalle vom Schmp. 101°C. Die Struktur und Reinheit der Verbindung wurde mit analytischen und spektroskopischen Daten gesichert.

Die übrigen, im Beispiel 3 genannten Verbindungen werden analog hergestellt.

### Beispiel 2

### Herstellung von 1-(Undecylamino)guanidin [H₂C11AG]

1,2 g 1-(Undecylidenamino)guanidin werden in einen Autoklaven gegeben und über einen Zeitraum von 12 h in Gegenwart von 0,1 g Palladium auf Aktivkohle (10 %) als Hydrierungskatalysator in 20 ml 100 prozentiger Essigsäure unter einem Wasserstoffdruck von 60 bar bei Zimmertemperatur hydriert. Danach wird der Katalysator abfiltriert und die farblose Lösung i.V. zur Trockne eingeengt.

Auf diesem Wege wird die Titelverbindung nach dem Umkristallisieren aus Essigester in Form farbloser Kristalle mit einem Schmelzbereich von 70 - 72°C in quantitativer Ausbeute isoliert.

### Beispiel 3

Die virostatischen Eigenschaften wurden durch in vitro Untersuchungen bestimmt. Folgende Virusstämme wurden eingesetzt:
Herpes Virus
Vesikular Stomatitis Virus
BVI 1

Zellkulturen (Affennierenzellen oder menschliche Fibrolasten werden mit Herpes infiziert und eine Reihe von Kulturen mit Medium versetzt, das verschiedene Konzentrationen der zu testenden Substanz enthält. Nach 24 Stunden wird die Konzentration der Virusnachkommenschaft im Zellkulturüberstand durch Plaqueassays bestimmt. Aus Dosis-Wirkungskurven wird die Konzentration der Substanz bestimmt, bei der die Virusvermehrung um 50 % gehemmt ist IC₅₀). Die erhaltenen Ergebnisse einiger Beispielsubstanzen sind in der nachfolgenden Tabelle aufgeführt.

| Substanz | IC₅₀ µM |
|---|---|
| 1-(Octyliden-amino)guanidin | 49,7 |
| 1-(Nonyliden-amino)guanidin | 29,0 |
| 1-(Decyliden-amino)guanidin | 28,9 |
| 1-Undecyliden-amino)guanidin | 6,8 |
| 1-(Dodecylidenamino)guanidin | 3,2 |

### Beispiel 4

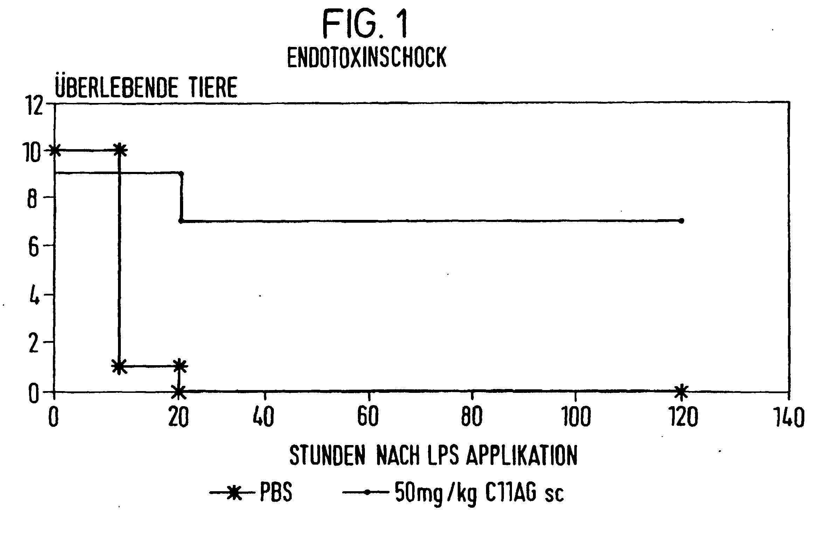

Der Schutz von Endotoxinschock durch C11AG wird durch Fig. 1 belegt:

Mäuse (Stamm NMRI/Nu, 8 Wochen alt, weiblich) erhielten je 0,2 mg Endotoxin aus E. coli (SIGMA, München) intraperitoneal verabreicht. Die 10 Kontrolltiere, die 0,2 ml 5 % Glukose subkutan verabreicht bekommen hatten, starben innerhalb von 24 Stunden. Neun Tieren wurde 30 Min vor der Endotoxinbehandlung 50 mglkg C11AG subkutan injiziert. Von dieser Gruppe starben nur 2 Tiere.

### Beispiel 5

### Hemmung der Collagen induzierten Arthritis in der Maus

Eine Autoimmunreaktion gegen Knorpelgewebe wurde durch Injektion von Collagen in DBA/I Mäuse erzeugt, wie beschrieben (Holmdahl, R. et al, Immunology, 65, 305 - 310, 1988) Je 10 Tiere dienten als Kontrolle, oder erhielten 50 mg/kg oder 100 mg/kg C11AG pro Tag oral. Die Verabreichung erfolgte über das Futter (Altromin, Pulverfutter), die Dosis wurde aus der täglichen Putteraufnahme berechnet. Die Symptome wurde für jede einzelne Pfote von 0,5 - 3 wie beschrieben [R. Holmdahl, et al., Immunology, 65, 305 - 310, (1988)] täglich bewertet. Die Summe der Symptome aller Tiere pro Gruppe - am Tag 7 nach der Booster-Injektion - wird durch die folgende Tabelle wiedergegeben:

| Behandlung | Summe der Symptome |
|---|---|
| Kontrolle | 0 |
| Collagen | 35 |
| Collagen/50 mg/kg C11AG | 4,5 |
| Collagen/100mg/kg C11AG | 1 |

20 Tage nach der Boosterinjektion wurden die Tiere getötet und die Gelenke histopathologisch befundet. Dabei ergibt sich folgendes Bild:

Bei allen unbehandelten Tieren wurden inflammatorische Prozesse festgestellt, bei den mit 50 und 100 mglkg C11AG behandelten Tieren und Kontrollen waren solche nicht feststellbar.

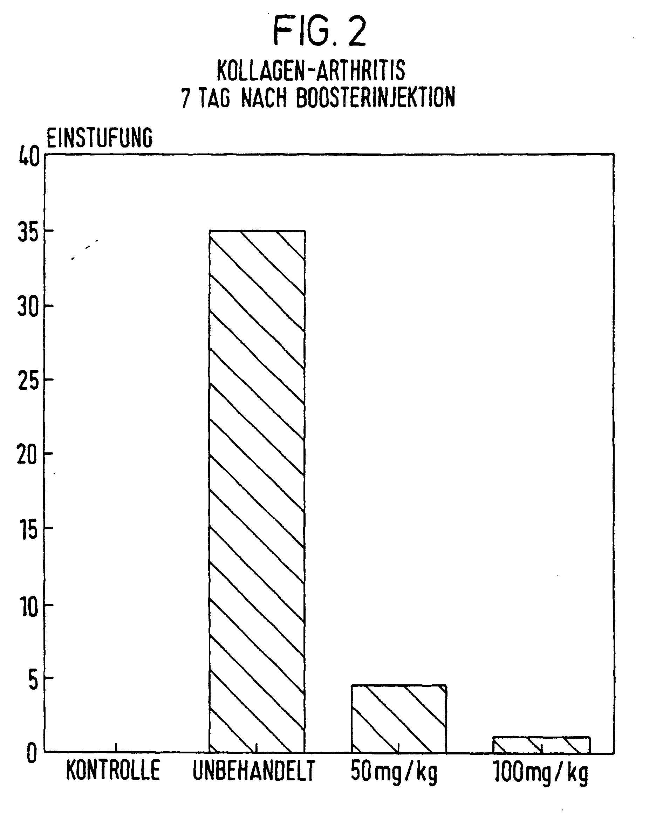

Die Ergebnisse, die sieben Tage nach der "Booster-Injektion" erhalten wurden, sind in Fig. 2 graphisch dargestellt.

### Beispiel 6

### Hemmung neutraler SMase

| Verbindung | neutrale SMase IC50[µM] |
|---|---|
| Octyliden-Aminoguanidin | 63 |
| Decyliden-Aminoguanidin | 44 |
| Undecyliden-Aminoguanidin | 8,2 |
| Dodecyliden-Aminoguanidin | 5,8 |

¹⁴C Sphingomyelin (10 µg/ml) wurde mit neutraler SMase (Membranfraktion aus Mäusehim, 10 µg Protein/Ansatz, isoliert [nach S.Gatt, Biochem. Biophys. Res. Commun. 68, 235-241 (1976)] in Anwesenheit verschiedener Konzentrationen der Testsubstanzen (für 2h bei 37°C 20 mM Tris, 1mM MgCl₂. pH 7,5) inkubiert. Anschließend wurden die Proben mit dem 5-fachen Volumen Chloroform/Methanol (1:1) extrahiert und der Gehalt an radioaktivem Phosphorylcholin in der wäßrigen Phase bestimmt Aus Dosis-Wirkungskurven wurde die IC₅₀ entnommen.

### Beispiel 7

### Hemmung der NO-Synthaseiduktion durch C11AG in Makrophagen

RAW Zellen (Mausmakrophagenlinie, Herkunft: American Type Culture Collection) wurden mit 10 ng/ml Endotoxin aus E coli (LPS) behandelt in Anwesenheit verschiedener Konzentrationen von C11AG. Nach 16 h wurde der Nitritgehalt im Kulturmedium nach beschriebener Methode [K. Tschaikowsky, M. Meisner, F. Schonhuber und E. Rugheimer, Br. J. Pharmacol. 113 (3): 664-8 (1994)] gemessen.

### Meßwerte:

| C11AG Konzentration [µg/ml] | OD 540 nm |
|---|---|
| 0 | 0,122 |
| 0,5 | 0,091 |
| 1 | 0,075 |
| 2 | 0,054 |
| 3 | 0,05 |
| 4 | 0,038 |

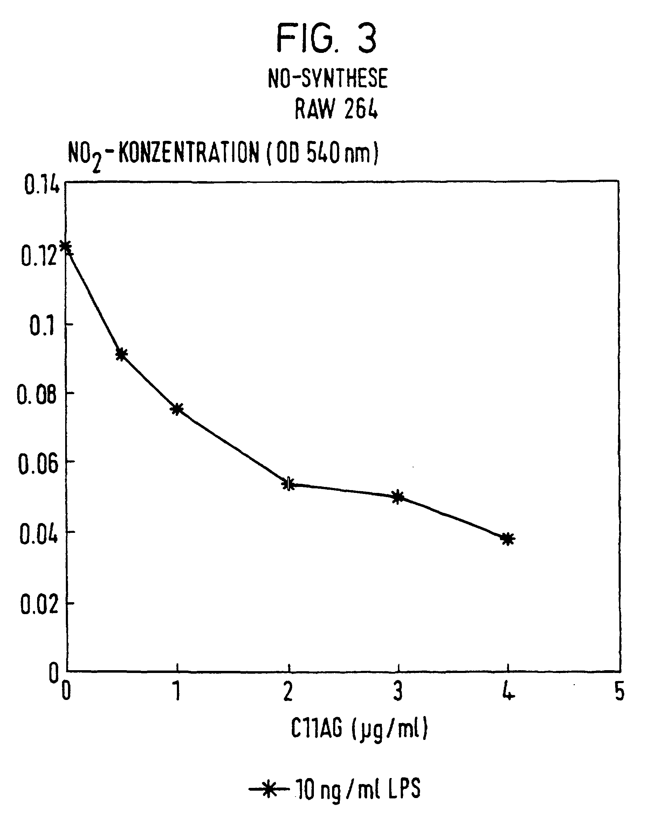

Die Hemmung der NO-Synthaseinduktion ist in Fig. 3 graphisch dargestellt; dort ist die NO₂-Konzentration [OD gemessen bei 540 nm] gegen die C11AG-Konzentration [µg/ml] für 10 nglml LPS aufgetragen.

### Beispiel 8

### C11AG-IC₅₀ Bestimmung saure und neutrale SMase

¹⁴C Sphingomyclin ((10 µg/ml) wurde mit neutraler SMase (Membranfraktion aus Mäusehirn, 10 µg Protein/Ansatz, isoliert nach Gatt, S. Biochem. Biophys. Res. Commun. 68, 235-241, 1976) oder mit saurer SMase (Mikrosomenfraktion aus Makrophagen 5 µg Protein/Ansatz isoliert nach Gatt, S. Biochem. Biophys. Res. Commun. 68, 235-241, 1976) in Anwesenheit verschiedener Konzentrationen der Testsubstanzen für 2h bei 37°C in 20mM Tris, 1mM MgCl₂, pH 7,5 (neutrale SMase) oder in 50 mM Natriumacetat, 1mM MgCl₂, pH 5,6 (saure SMase) inkubiert. Anschließend wurden die Proben mit dem 5-fachen Volumen Chloroform/Methanol (1:1) extrahiert und der Gehalt an radioaktivem Phosphorylcholin in der wäßrigen Phase bestimmt. Die Phosphorylcholinfreisetzung in den unbehandelten Ansätzen entspricht 100 % Enzymaktivität.

### Meßwerte:

| C11AG Konzentration [µg/ml] [%] | nSMase-Aktivität [%] | sSMase-Aktivität |
|---|---|---|
| 0 | 100 | 100 |
| 1 | 61 | 101 |
| 10 | 18 | 102 |
| 100 | 0 | 31 |

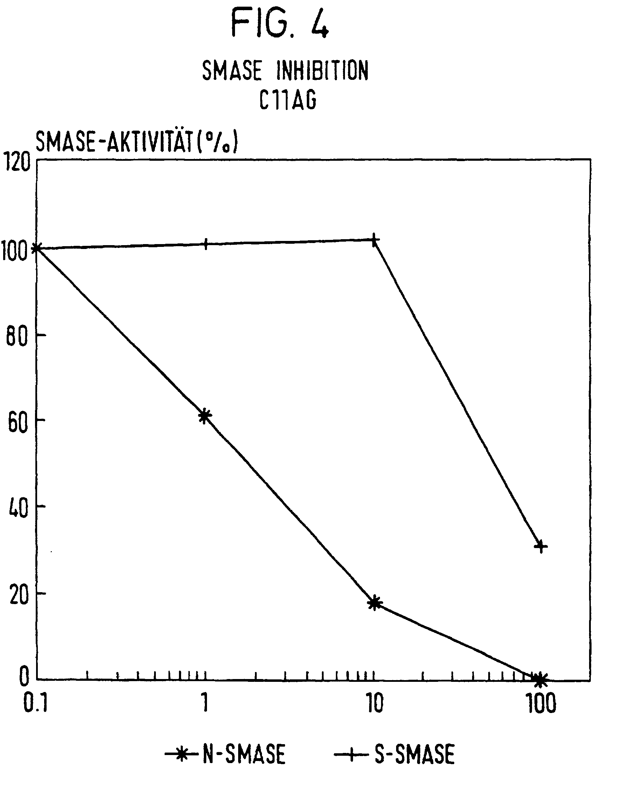

Fig. 4. zeigt in einfach-logarithmischer Darstellungsweise die Sphingomyelinase Inhibierung für neutrale und saure Sphingomyelinase [in %] in Abhängigkeit von der C11AG-Konzentration [µg/ml].

### Beispiel 9

### Hemmung des Wachstums von Papillomen

Mastomys natalensis mit von einem Popillomavirus ausgelösten Papillomen [siehe E. Amtmann und K. Wayss: The Mastomys natalensis Papillomavirus, in: P. Salzman und P. Howley (Eds.): The Papovaviridae, Vol. 2. Plenum Publishing Corporation, (1987)] erhielten Futter, das verschiedene Mengen an C11AG enthielt. Der Futterverbrauch wurde gemessen und daraus die tägliche, orale C11AG-Dosis errechnet. Die Größe der Papillome wurde mittels Schublehre in zwei Dimensionen gemessen und das relative Wachstum errechnet. Pro Dosis wurden 10 Tiere behandelt.

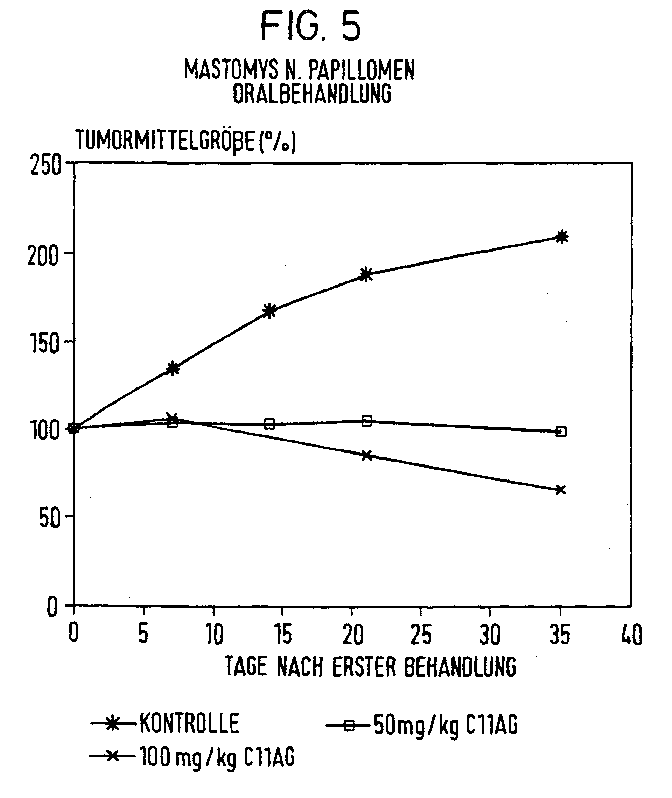

In Fig. 5 ist die mittlere Tumorgröße in Abhängigkeit von der Behandlungsdauer für verschiedene Dosierungen an C11AG graphisch dargestellt. Dabei gibt Kurve A das Tumorwachstum der Kontrolltiere wieder. Kurve B zeigt den Größenverlauf für eine Dosierug von 50mg/kg C11AG und Kurve C zeigt den entsprechenden Verlauf für 100 mg/kg C11AG.

### Beispiel 10

### Hydriertes C11AG-IC₅₀: Bestimmung saure und neutrale SMase

¹⁴C Sphingomyelin (10 µg/ml) wurde mit neutraler SMase (Membranfraktion aus Mäusehirn, 10 µg Protein/Ansatz [isoliert nach Gatt, S. Biochem. Biophys. Res. Commun. 68, 235-241, (1976)] oder mit saurer SMase (Mikrosomenfraktion aus Makrophagen 5 µg Protein/Ansatz, isoliert [nach S. Gatt,. Biochem. Biophys. Res. Commun. 68, 235-241, (1976)] in Anwesenheit verschiedener Konzentrationen der Testsubstanzen für 2h bei 37°C in 20mM Tris, 1mM MgCl₂, pH 7,5 (neutrale SMase) oder in 50mM Natriumacetat, 1mM MgCl₂, pH 5,6 (saure SMase) inkubiert. Anschließend wurden die Proben mit dem 5-fachen Volumen Chloroform/Methanol (1:1) extrahiert und der Gehalt an radioaktivem Phosphorylcholin in der wäßrigen Phase bestimmt. Die Phosphorylcholinfreisetzung in den unbehandelten Ansätzen entspricht 100 %.

### Meßwerte:

| H₂C11AG Konzentration [µg/ml] [%] | nSMase-Aktivität [%] | sSMase-Aktivität |
|---|---|---|
| 0 | 100 | 100 |
| 1 | 73 | 100 |
| 10 | 22 | 97 |
| 100 | 2 | 32 |

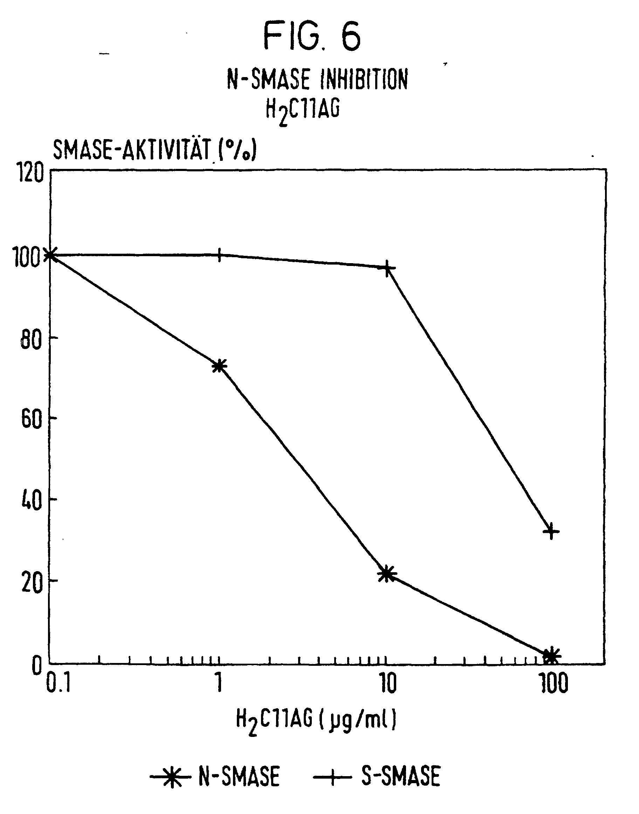

Fig. 6 zeigt in einfach-logarithmischer Darstellungsweise die Sphingomyelinase Inhibierung für neutrale - Kurve A - und saure Sphingomyelinase [in %] - Kurve B - in Abhängikeit von der H₂C11AG-Konzentration [µg/ml].

### Beispiel 11

### Prävention von letalem Endotoxinschock in der Maus durch H₂C11AG

Je 10 Mäuse des Stammes Balb C (ca. 8 Wochen alten) erhielten 0,7 mg Endotoxin aus E coli (in 0,2 ml isotonischer Kochsalzlösung) i.p. gespritzt. 10 Tiere bekamen 2h vor der LPS-Behandlung 100 mg/kg Körpergewicht H₂C11AG (in Aqua bidest gelöst) mittels Schlundsonde verabreicht. Die Kontrolltiere erhielten Waser. Die überlebenden Tiere wurden 12 Tage beobachtet.

Ergebnis: Kontrolle 2 Überlebende (20 %), 100 mg/kg H₂C11AG (hydriertes C11AG) 9 Überlebende (90 %).

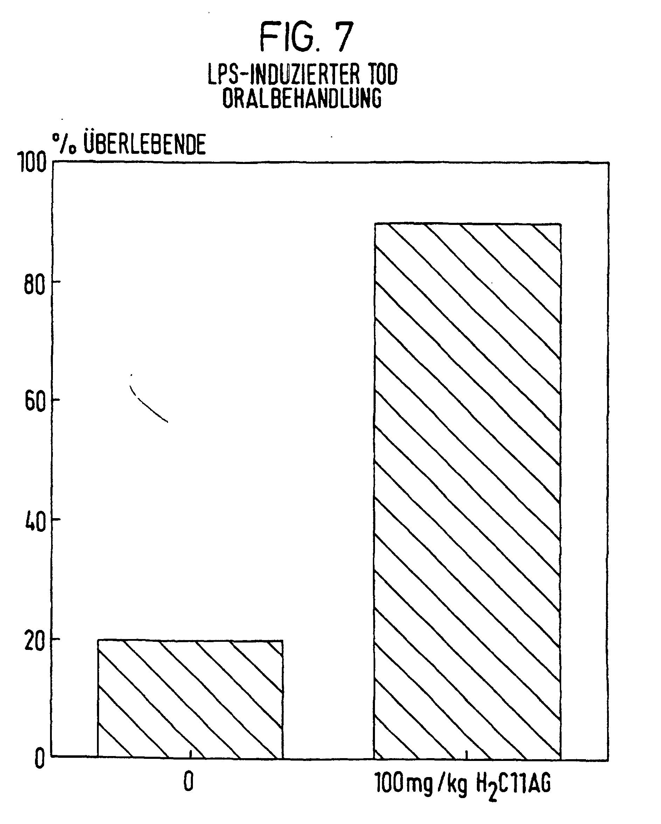

Fig. 7 zeigt die Überlebensrate von unbehandelten Versuchtieren (A) im Vergelich zu denjenigen, die - wie oben beschrieben - mit einer Dosis von 100 mg H₂C11AG behandelt wurden.

Um die in der Anmeldung verwendete Nomenklatur zu erläutern, werden die Strukturen einiger genannter Verbindungen angegeben:

## Patentansprüche

1. Chemische Verbindung der Formel: 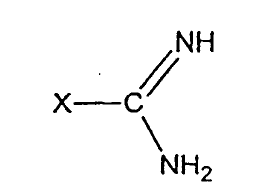 wobei X -NH-NH-CH₂R₁ symbolisiert und R₁ ein C₈ bis C₂₀ - Alkyl symbolisiert, entweder verzweigt oder unverzweigt; wahlweise in Form der einzelnen optischen Isomere, Mischungen der einzelnen Isomere oder Racemate, Tautomere wie z.B. geometrische Isomere z.B. cis/trans Isomere, ferner in Form der freien Basen oder entsprechenden Salze, die durch Zugabe pharmakologisch anwendbarer Säuren entstehen.

2. Chemische Verbindung nach Anspruch 1, wobei R₁ eine unverzweigte Decylgruppe symbolisiert.

3. Chemische Verbindung nach Anspruch 1 oder Anspruch 2, wobei es sich um 1-(Undecylamino)guanidin (H₂C11AG) handelt.

4. Pharmazeutische Zusammensetzung, die eine chemischen Verbindung mit folgender Formel erhält: 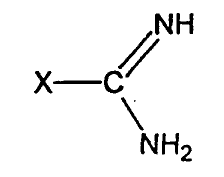 wobei X -NH-NH-CH₂R₁ oder -NH-N=CHR, symbolisiert und R₁ ein C₈ bis C₂₀ - Alkyl symbolisiert, entweder verzweigt oder unverzweigt; wahlweise in Form der einzelnen optischen Isomere, Mischungen der einzelnen Isomere oder Racemate, Tautomere wie z.B. geometrische Isomere z.B. cis/trans Isomere, femer in Form der freien Basen oder entsprechenden Salze, die durch Zugabe pharmazeutisch verwendbarer Säuren entstehen, gemischt mit pharmazeutisch verwendbaren Bindemitteln und Trägerstoffen.

5. Pharmazeutische Zusammensetzung nach Anspruch 4, wobei X -NH-N=CH-CH₂-CH₂-(CH₂)₇-CH₃ symbolisiert.

6. Chemische Verbindung der Formel: 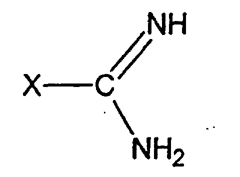 wobei X -NH-NH-CH₂R₁ oder -NH-N=CHR₁ symbolisiert und R, ein C₈ bis C₂₀ - Alkyl symbolisiert, entweder verzweigt oder unverzweigt; wahlweise in Form der einzelnen optischen Isomere, Mischungen der einzelnen Isomere oder Racemate, Tautomere wie z.B. geometrische Isomere z.B. cis/trans Isomere, ferner in Form der freien Basen zur Verwendung als Medikament.

7. Chemische Verbindung nach Anspruch 6, wobei X -NH-N=CH-CH₂-CH₂-(CH₂)₇-CH₃ symbolisiert.

8. Verbindung nach Anspruch 6 oder Anspruch 7, wobei das genannte Medikament Sphingomyelinase hemmt.

9. Verwendung einer Verbindung der Formel: 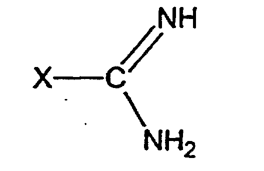 wobei X -NH-NH-CH₂R₁ oder -NH-N=CHR₁ symbolisiert und R₁ ein C₈ bis C₂₀ - Alkyl symbolisiert, entweder verzweigt oder unverzweigt; wahlweise in Form der einzelnen optischen Isomere, Mischungen der einzelnen Isomere oder Racemate, Tautomere wie z.B. geometrische Isomere z.B. cis/trans Isomere, ferner in Form der freien Basen zur Herstellung eines Medikamentes zur Hemmung der Sphingomyelinase.

10. Verwendung nach Anspruch 9, wobei X-NH-N=CH-CH₂-CH₂-(CH₂)₇-CH₃ symbolisiert.

11. Verwendung nach Anspruch 9 oder Anspruch 10, wobei die genannte Sphingomyelinase neutrale Sphingomyelinase ist.

12. Verwendung nach Anspruch 9 oder Anspruch 10 oder Anspruch 11, wobei das genannte Medikament für die Behandlung von entzündlichen Erkrankungen und Autoimmunerkrankungen eingesetzt wird.

13. Verwendung nach Anspruch 12, wobei die genannten entzündlichen Erkrankungen und Autoimmunerkrankungen aus folgender Gruppe stammen: chronische Polyarthritis, seronegative Arthropathie, Emphysembronchitis, chronische obstruktive Atemwegserkrankungen, Osteoarthritis, entzündliche Darmerkrankungen, Morbus Crohn , systemische Lupus Erythematose, Iridozyklitis, Uveitis, Optikusneuritis, idiopathische Lungenfibrose, systemische Vasculitis/Wegener Granulomatose, Sarkoidose und Orchitis/Vasektomie Reversionsverfahren.

14. Verwendung nach Anspruch 9 oder Anspruch 10 oder Anspruch 11, wobei das genannte Medikament für die Behandlung von kardiovaskulären Erkrankungen eingesetzt wird.

15. Verwendung nach Anspruch 14, wobei genannte kardiovaskuläre Erkrankungen aus folgender Gruppe stammen: cardiac stun syndrome, Myokardinfarkt, Stauungsinsuffizienz und Arteriosklerose.

16. Verwendung nach Anspruch 9 oder Anspruch 10 oder Anspruch 11, wobei das genannte Medikament für die Behandlung von Infektionskrankheiten eingesetzt wird.

17. Verwendung nach Anspruch 16, wobei genannte Infektionskrankheiten aus folgender Gruppe stammen: Papilloma-Virusinfektion, Infektionen mit dem Herpesvirus, Infektionen mit HIV/HIV Neuropathie, Meningitis, Hepatitis, septische Arthritis, Peritonitis, Pneunomia, Bronchitis, Epiglottitis. Escheria coli 0157:H7 Infektion, hämolytisches Urämie-Syndrom, thrombolytische thrombozytopenische Purpura, Malaria, Dengue Fieber/hämorrhagisches Fieber, Leishmaniase, Lepra, toxisches Schocksyndrom, Streptokokken-Myositis, Gasgangrän, Tuberkuloseinfektionen mit dem Mycobakterium, intrazelluläre Infektionen mit Mycobacterium avium, Pneumocyetose, Entzündungen des Unterleibs, Orchitis/Epidydimitis, Legionärskrankheit, Lyme-Krankheit, Infektionen mit dem Influenza A Virus, durch den Eppstein-Barr Virus verursachte Infektionen, viral verursachtes haemaphagocytisches Syndrom, virale Encephalitis/aseptische Menginitis.

18. Verwendung nach Anspruch 9 oder Anspruch 10 oder Anspruch 11, wobei das genannte Medikament für die Behandlung von malignen Erkrankungen und zur Tumortherapie eingesetzt wird.

19. Verwendung nach Anspruch 18, wobei das genannte Medikament in der Tumortherapie in Verbindung mit Chemotherapie, Radiotherapie und Zytokinbehandlung verwendet wird, wie z:B.: TNF-α Behandlung, zur Behandlung von Sarkomen, Karzinomen, Leukämien,
ALL (akute Lymphoblastenleukämie)
AML (akute Myeloblastenleukämie)
CML (chronische myeloische Leukämie)
CLL (chronische lymphatische Leukämie
kleinzelliges und nicht kleinzelliges Bronchialkarzinom
Brustkrebs
Pflasterzell-, Plattenepithelkarzinom
Hodgkin Krankheit, nicht Hodgkin Lymphom
multiples Melanom
Kaposi Sarkom
kolorektales Karzinom
nasopharyngeales Karzinom
maligne Histiozytose
paraneoplastisches Syndrom/maligne Hyperualkämie

20. Verfahren zur Herstellung einer Verbindung gemäß Anspruch 1, umfassend eine erste Produktionsstufe, bei der ein Aldehyd oder ein Keton mit der allgemeinen Formel R₁CHO mit einem Aminoguanidin zur Reaktion gebracht wird und das Reaktionsprodukt isoliert wird und wahlweise das entsprechende Salz durch Zugabe einer pharmakologisch anwendbaren Säure gebildet wird.

21. Verfahren nach Anspruch 20, weiter umfassend einen zweiten Verfahrensschritt, bei dem die Iminfunktion, die beim ersten Verfahrensschritt entsteht, durch Anwendung eines Hydrierungskatalysators unter erhöhtem Wasserstoffdruck reduziert wird.

## Claims

1. A compound having the formula: 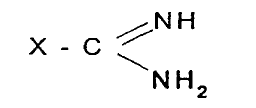 wherein X denotes -NH-NH-CH₂R₁ and R₁ denotes C₈ to C₂₀ - Alkyl, either branched or unbranched; and optionally in the form of the individual optical isomers, mixtures of the individual isomers or racemates, tautomers such as geometrical isomers e.g. cis/trans isomers, as well as in the form of the free bases or corresponding acid addition salts with pharmacologically acceptable acids.

2. A compound according to claim 1 wherein R₁ denotes an unbranched decyl group.

3. A compound according to claim 1 or claim 2 being 1-(undecylamino)guanidine (H₂C11AG).

4. A pharmaceutical composition comprising a compound having the formula: 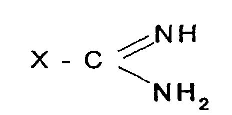 wherein X denotes -NH-NH-CH₂R₁ or -NH-N=CHR₁, and R₁ denotes C₈ to C₂₀ -Alkyl, either branched or unbranched; and optionally in the form of the individual optical isomers, mixtures of the individual isomers or racemates, tautomers such as geometrical isomers e.g. cis/trans isomers, as well as in the form of the free bases and the acid addition salts thereof with pharmaceutically acceptable acids together with pharmaceutically acceptable excipients and carriers.

5. A pharmaceutical composition according to claim 5, wherein X denotes -NR-N=CH-CH₂-CH₂-(CR₂)₇-CH₃.

6. A compound having the formula: 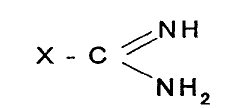 wherein X denotes -NH-NH-CH₂R₁ or -NH-N=CHR₁, and R₁ denotes C₈ to C₂₀ -Alkyl, either branched or unbranched; and optionally in the form of the individual optical isomers, mixtures of the individual isomers or racemates, tautomers such as geometrical isomers e.g. cis/trans isomers, as well as in the form of the free bases for use as a medicament.

7. A compound according to claim 6, wherein X denotes -NH-N=CH-CH₂-CH₂-(CH₂)₇-CH₃.

8. A compound according to claim 6 or claim 7 wherein said medicament inhibites sphingomyelinase.

9. Use of a compound having the formula: 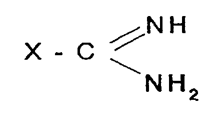 wherein X denotes -NH-NH-CH₂R₁ or -NH-N=CHR₁, and R₁ denotes C₈ to C₂₀ -Alkyl, either branched or unbranched; and optionally in the form of the individual optical isomers, mixtures of the individual isomers or racemates, tautomers such as geometrical isomers e.g. cis/trans isomers, as well as in the form of the free bases in the preparation of a medicament for inhibiting sphingomyelinase.

10. Use according to claim 9, wherein X denotes -NH-N=CH-CH₂-CH₂-(CH₂)₇-CH₃.

11. Use according to claim 9 or claim 10 wherein said sphingomyelinase is neutral sphingomyelinase.

12. Use according to one of claim 9 and 10 and 11 wherein said medicament is for the treatment of inflammatory diseases and autoimmune diseases.

13. Use according to claim 12 wherein said inflammatory diseases and autoimmune diseases are selected from rheumatoid arthritis, seronegative arthropathy, emphysema bronchitis, chronic obstructive pulmonary disease (COPD), osteoarthrits, inflammatory bowel disease, Crohn's disease, systemic lupus erythematosis, iridocyclitis, uveitis, optic neuritis, idiopathic pulmonary fibrosis, systemic vasculitis/Wegner's granulomatosis, sarcoidosis and orchitis/vasectomy reversal procedures.

14. Use according to one of claims 9 and 10 and 11 wherein said medicament is for the treatment of cardiovascular diseases.

15. Use according to claim 14 wherein said cardiovascular diseases are selected from cardiac stun syndrome, myocardial infarction, congestive heart failure and arteriosclerosis.

16. Use according to one of claims 9 and 10 and 11 wherein said medicament is for treating infectious diseases.

17. Use according to claim 16 wherein said infectious diseases are selected from papilloma virus infectious, herpes virus infections, HIV infection/HIV neuropathology, meningitis, hepatitis, septic arthritis, peritonitis, pneumonia, bronchitis, epiglottitis, E. coli 0157:H7 infection, haemolytic uremia syndrome/thrombolytic thrombocytopenic purpura, malaria, Dengue haemorrhagic fever, leishmaniasis, leprosy, toxic shock syndrome, *Streptococcal* myositis, gas gangrene, mycobacterium tuberculosis infections, mycobacterium avium intracellular infections, pneumocyetosis, pelvic inflammatory disease, orchitis/epidydimitis, legionella, lyme disease, influenza A virus infections, infections caused by Epstein-Barr virus, viral associated haemaphagocytic syndrome, viral encephalitis/aseptic meningitis.

18. Use according to one of claims 9 and 10 and 11 wherein said medicament is for treating malignant diseases and for tumour therapy.

19. Use according to claim 18 wherein said medicament is for use in tumour therapy in conjunction with chemotherapy, radiotherapy and cytokine treatment, such as, for example: TNF-α treatment, for treating sarcomas, carcinomas, leukaemias
ALL
AML
CML
CLL
small cell and non-small cell bronchial carcinoma breast cancer
squamous cell carcinoma
Hodgkin's disease, non-Hodgkin's lymphoma
multiple melanoma
Kaposi's sarcoma
colorectal carcinoma
nasopharyngeal carcinoma
malignant histiocytosis
paraneoplastic syndrome/hyperualcaemia of malignancy.

20. A process for preparing a compound according to claim 1 comprising a first step of reacting an aldehyde or a ketone having the general formula R₁CHO with an aminoguanidine and isolating the reaction product and optionally forming the corresponding acid addition salt with a pharmacological acceptable acid.

21. A process according to claim 20 further comprising a second step of reducing the imine function resulting from the first step in the presence of a hydrogenation catalyst under elevated hydrogen pressure.

## Revendications

1. Composé chimique ayant pour formule: 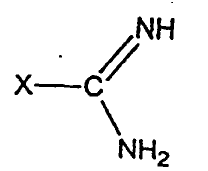 où X désigne -NH-NH-CH₂R₁ et R₁ désigne alkyle C₈ à C₂₀ , soit ramifié ou non ramifié; et éventuellement sous la forme d'isomères optiques individuels, mélanges des isomères individuels ou racémates, tautomères comme des isomères géométriques e.g. isomères cis/trans, ainsi que sous la forme des bases libres ou sels correspondants d'addition d'acide avec des acides pharmacologiquement acceptables.

2. Composé chimique selon la revendication 1 où R₁ désigne un groupe décyle non ramifié.

3. Composé chimique selon la revendication 1 ou la revendication 2 où il s'agit de 1-(undécylamino)guanidine (H₂C11AG).

4. Composition pharmaceutique comprenant un composé ayant pour formule: 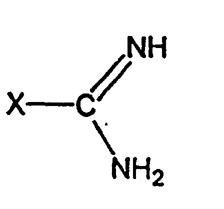 où X désigne -NH-NH-CH₂R₁ ou -NH-N=CHR₁, et R₁ désigne un alkyle C₈ à C_{20,} soit ramifié ou non ramifié; éventuellement sous la forme des isomères optiques individuels, de mélanges des isomères individuels ou racémates, de tautomères comme des isomères géométriques e.g. isomères cis/trans, ainsi que sous la forme des bases libres et de leurs sels d'addition d'acide avec des acides pharmaceutiquement acceptables ainsi que des excipients et supports pharmaceutiquement acceptables.

5. Composition pharmaceutique selon la revendication 4, où X désigne -NH-N=CH-CH₂-CH₂-(CH₂)₇-CH₃.

6. Composé ayant la formule: 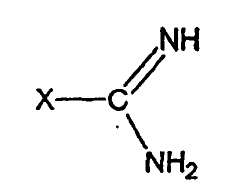 où X désigne -NH-NH-CH₂R₁ ou -NH-N=CHR₁ et R₁ désigne alkyle C₈ à C₂₀, soit ramifié ou non ramifié; et facultativement sous la forme des isomères optiques individuels, de mélanges des isomères individuels ou racémates, de tautomères, comme des isomères géométriques e.g. isomères cis/trans, ainsi que sous la forme des bases libres pour une utilisation comme médicament.

7. Composé selon la revendication 6, où X désigne -NH-N=CH-CH₂-CH₂- (CH₂)₇-CH₃.

8. Composé selon la revendication 6 ou la revendication 7 où ledit médicament inhibe la sphingomyélinase.

9. Utilisation d'un composé ayant la formule: 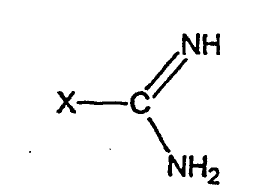 où X désigne -NH-NH-CH₂R₁ ou -NH-N=CHR₁ et R₁ désigne alkyle C₈ à C₂₀ soit ramifié ou non ramifié; et facultativement sous la forme des isomères optiques individuels, mélanges des isomères individuels ou racémates, tautomères comme les isomères géométriques e.g. isomères cis/trans, ainsi que sous la forme des bases libres dans la préparation d'un médicament pour inhiber la sphingomyélinase.

10. Utilisation selon la revendication 9, où X désigne -NH-N=CH-CH₂-CH₂- (CH₂)₇-CH₃.

11. Utilisation selon la revendication 9 ou la revendication 10 où ladite sphingomyélinase est une sphingomyélinase neutre.

12. Utilisation selon l'une quelconque des revendications 9 et 10 et 11 où ledit médicament est pour le traitement des maladies inflammatoires et des maladies auto-immunes.

13. Utilisation selon la revendication 12 où lesdites maladies inflammatoires et maladies autoimmunes sont sélectionnées parmi l'arthrite rhumatoïde, l'arthropathie séronégative, la bronchite emphysémateuse, la maladie pulmonaire obstructive chronique (COPD), l'ostéoarthrite, une maladie inflammatoire des intestins, la maladie de Crohn, le lupus érythématoseux généralisé, l'iridocyclite, l'uvéite, la neurite optique, la fibrose idiopathique pulmonaire, la vasculite généralisée/granulomatose de Wegner, la sarcoidose et les processus d'inversion de vasectomie/orchite.

14. Utilisation selon l'une quelconque des revendications 9 et 10 et 11 où ledit médicament est pour le traitement des maladies cardiovasculaires.

15. Utilisation selon la revendication 14 où lesdites maladies cardiovasculaires sont sélectionnées parmi le syndrome d'étourdissement cardiaque, l'infarctus du myocarde, l'asystolie congestive et l'artériosclérose.

16. Utilisation selon l'une quelconque des revendications 9 ou 10 ou 11 où ledit médicament est pour le traitement des maladies infectieuses.

17. Utilisation selon la revendication 16 où lesdites maladies infectieuses sont sélectionnées parmi des infections par le virus du papillome, des infections par le virus de l'herpès, des infections par VIH/neuropathologie VIH, la méningite, l'hépatite, l'arthrite septique, la péritonite, la pneumonie, la bronchite, l'épiglottite, une infection par E. coli 0157:H7, le syndrome d'urémie hémolytique/purpura thrombocytopénique thrombolytique, la malaria, la fièvre hémorragique de Dengue, la leishmania, la lèpre, le syndrome du choc toxique, la mycosite due aux Streptocoques, la gangrène gazeuse, les infections de tuberculose mycobactérienne, les infections intracellulaires aviennes mycobactériennes, la pneumocyétose, la maladie inflammatoire pelvienne, 1'orchite/épidydimite, la légionellose, la maladie de lyme, des infections par le virus de la grippe A, des infections provoquées par le virus de Epstein-Barr, le syndrome hémaphagocytaire associé viral, l'encéphalite virale/méningite aseptique.

18. Utilisation selon l'une quelconque des revendications 9 et 10 et 11 où ledit médicament est pour le traitement des maladies malignes et pour la thérapie des tumeurs.

19. Utilisation selon la revendication 18 où ledit médicament est pour une utilisation dans la thérapie des tumeurs en conjonction avec la chimiothérapie, la radiothérapie et le traitement avec les cytokines, comme, par exemple: traitement par TNF-α, pour le traitement des sarcomes, des carcinomes, des leucémies
ALL
AML
CML
CLL
Carcinome bronchique des petites cellules et des cellules non petites
Cancer du sein
carcinome des cellules squameuses
maladie de Hodgkin, lymphome non Hodgkin
mélanome multiple
sarcome de Kaposi
carcinome colorectal
carcinome nasopharyngé
histiocytose maligne
syndrome paranéoplastique/hyperualcémie de malignité.

20. Procédé de préparation d'un composé selon la revendication 1 comprenant une première étape de réaction d'un aldéhyde ou d'une cétone ayant la formule générale R₁CHO avec une aminoguanidine et d'isolement du produit réactionnel et facultativement de la formation du sel d'addition d'acide correspondant avec un acide pharmacologique acceptable.

21. Procédé selon la revendication 20 comprenant de plus une seconde étape de réduire la fonction imine résultant de la première étape en présence d'un catalyseur d'hydrogénation sous une pression élevée d'hydrogène.
